(19) Europäisches Patentamt / European Patent Office / Office européen des brevets

(11) **EP 3 126 514 B1**

(12) **EUROPEAN PATENT SPECIFICATION**

(45) Date of publication and mention of the grant of the patent:
**28.02.2018 Bulletin 2018/09**

(21) Application number: **15715207.5**

(22) Date of filing: **01.04.2015**

(51) Int Cl.:
*C12Q 1/68* (2018.01)    *G01N 33/53* (2006.01)

(86) International application number:
**PCT/EP2015/057238**

(87) International publication number:
**WO 2015/150482 (08.10.2015 Gazette 2015/40)**

(54) **A COMBINATION PRODUCT COMPRISING A NUCLEIC ACID STRUCTURE AND A SINGLE-STRANDED NUCLEIC ACID MOLECULE**

KOMBINATIONSPRODUKT MIT EINER NUKLEINSÄURESTRUKTUR UND EINEM EINSTRÄNGIGEN NUKLEINSÄUREMOLEKÜL

PRODUIT DE COMBINAISON COMPRENANT UNE STRUCTURE D'ACIDE NUCLÉIQUE ET UNE MOLÉCULE D'ACIDE NUCLÉIQUE SIMPLE BRIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.04.2014 GB 201405919**

(43) Date of publication of application:
**08.02.2017 Bulletin 2017/06**

(73) Proprietor: **Dynamic Biosensors GmbH 82152 Martinsried/Planegg (DE)**

(72) Inventors:
 • **PAUL, Hampel**
   **82110 Germering (DE)**
 • **ULRICH, Rant**
   **80807 München (DE)**

(74) Representative: **Lasar, Andrea Gisela Maiwald Patentanwalts GmbH Elisenhof Elisenstraße 3 80335 München (DE)**

(56) References cited:
**WO-A1-2004/013354      WO-A1-2008/122088
US-A1- 2003 219 803      US-B1- 6 177 555**

 • HUI DU ET AL: "Sensitivity and Specificity of Metal Surface-Immobilized "Molecular Beacon" Biosensors", JOURNAL OF THE AMERICAN CHEMICAL SOCIETY, vol. 127, no. 21, 2005, pages 7932-7940, XP055194835, ISSN: 0002-7863, DOI: 10.1021/ja042482a
 • Natalia E Broude: "Molecular Beacons and Other Hairpin Probes" In: "Encyclopedia of Diagnostic Genomics and Proteomics", 2005, XP055194756, pages 846-850, DOI: 10.1081/E-EDGP, figure 1
 • Y. ZHAO ET AL: "Rapid Real-Time Nucleic Acid Sequence-Based Amplification-Molecular Beacon Platform To Detect Fungal and Bacterial Bloodstream Infections", JOURNAL OF CLINICAL MICROBIOLOGY, vol. 47, no. 7, 29 April 2009 (2009-04-29) , pages 2067-2078, XP055138438, ISSN: 0095-1137, DOI: 10.1128/JCM.02230-08
 • IN-HEE LEE ET AL: "Non-linear molecular pattern classification using molecular beacons with multiple targets", BIOSYSTEMS, vol. 114, no. 3, 2013, pages 206-213, XP055195032, ISSN: 0303-2647, DOI: 10.1016/j.biosystems.2013.05.008
 • JELENA KNEZEVIC ET AL: "Quantitation of Affinity, Avidity, and Binding Kinetics of Protein Analytes with a Dynamically Switchable Biosurface", JOURNAL OF THE AMERICAN CHEMICAL SOCIETY, vol. 134, no. 37, 19 September 2012 (2012-09-19), pages 15225-15228, XP055194363, ISSN: 0002-7863, DOI: 10.1021/ja3061276 cited in the application

• **REBECCA MEYER ET AL: "Advances in DNA-directed immobilization", CURRENT OPINION IN CHEMICAL BIOLOGY, vol. 18, 21 November 2013 (2013-11-21), pages 8-15, XP055194719, ISSN: 1367-5931, DOI: 10.1016/j.cbpa.2013.10.023**

SEQUENCE LISTING

<110> Dynamic Biosensors GmbH

<120> A combination product comprising a nucleic acid structure and a
single-stranded nucleic acid molecule

<130> D 7504-WO

<160> 9

<170> PatentIn version 3.5

<210> 1
<211> 48
<212> DNA
<213> artificial

<220>
<223> single-stranded DNA

<400> 1
tagtcgtaag ctgatatggc tgattagtcg gaagcatcga acgctgat        48

<210> 2
<211> 90
<212> DNA
<213> artificial

<220>
<223> slingshot sequence

<400> 2

```
tagtcgcccg ctgatatggc tgattcgtcg tttttgcagc tggtgtcgag acgggttttt        60

cgacgaatca gccatatcag cgggcgacta        90
```

<210> 3
<211> 20
<212> DNA
<213> artificial

<220>
<223> spacer bar

<400> 3
cccgtctcga caccagctgc        20

<210> 4
<211> 48
<212> DNA
<213> artificial

<220>
<223> handle 1

<400> 4
tagtcgtaag ctgatatggc tgattagtcg gaagcatcga acgctgat        48

<210> 5
<211> 48
<212> DNA
<213> artificial

<220>
<223> handle 2

<400> 5
gtgtgaaccc tccaacaaag gtagcatttg ccagctctcg tgatgcag        48

<210> 6
<211> 96
<212> DNA
<213> artificial

<220>
<223> immobilization connector

<400> 6

```
ctgcatcacg agagctggca aatgctacct ttgttggagg gttcacacat cagcgttcga        60

tgcttccgac taatcagcca tatcagctta cgacta        96
```

<210> 7
<211> 119
<212> DNA
<213> artificial

<220>
<223> connector strand

<400> 7

```
atcagcgttc gatgcttccg actaatcagc catatcagct tacgactatg gaccctaaga          60

gactggtacg tctgcatcac gagagctggc aaatgctacc tttgttggag ggttcacac          119
```

<210> 8
<211> 10
<212> DNA
<213> artificial

<220>
<223> spacer 1

<400> 8
cgtaccagtc          10

<210> 9
<211> 11
<212> DNA
<213> artificial

<220>
<223> spacer 2

<400> 9
tcttagggtc c          11

**Description**

**FIELD OF THE INVENTION**

The present invention relates to a combination product which can be used to detect and/or characterize interactions between two molecules, in particular two proteins and most particular between an antigen and an antibody. The combination product comprises a nucleic acid structure comprising a double-stranded part and a single-stranded part which is capable of hybridizing to another single-stranded nucleic acid molecule which is also part of the combination product.

**BACKGROUND OF THE INVENTION**

In recent years, the use of therapeutic antibodies for the treatment of diseases such as cancer and autoimmune diseases has become increasingly important. In the development of these antibodies a thorough analysis of their binding characteristics and their interaction with their binding target is necessary.

One commonly employed method to characterize the interaction of an antibody with its target is surface plasmon resonance (SPR) in which one interactant of an interacting pair is immobilized on a gold-coated glass slide which is mounted on a prism and the other interactant is induced to flow across this surface in an aqueous buffer solution. When light passes through the prism and onto the gold surface at angles and wavelengths near the so-called "surface plasmon resonance" condition, the optical reflectivity of the gold changes when biomolecules are bound to the gold surface. By monitoring this reflectivity change the extent of interaction between the interactant on the gold surface and its binding partner in the aqueous buffer solution can be detected and quantified (Vancott (1999) Methods Mol. Med. 17: 273-282; Hearty et al. (2012) Methods Mol. Biol. 907: 411-442).

Another recently developed technique is the "switchSENSE" technology which is based on the electrical actuation of nucleic acid double strands to which an interactant is attached at high frequency on microelectrodes. The orientation of the nucleic acid double strands is then monitored by time-resolved single photon counting. If an analyte binds to the interactant attached to the nucleic acid double strand, the switching dynamics of the double strand is slowed down in a characteristic, detectable way (Rant et al., Nano Letters, 2009, Vol. 9, 1290 - 1295; Knezevic et al. (2012) J. Am. Chem. Soc. 134(37): 15225-15228; Langer et al. (2013) Nature Communications 4: 2099).

In the above methods using surface biosensors the interactant which is immobilized on the biosensor surface is typically randomly distributed, leading to differences in the local density of the interactant on the surface. Due to these differences the spatial arrangement on the surface of interactants such as antigens which bind to an antibody to be analyzed cannot be controlled. Consequently, when bivalent or bispecific antibodies are analyzed, a certain percentage of the antibodies will bind the antigen via only one Fab arm, if at the binding site no second antigen is available for binding, while another percentage of the antibodies will bind the antigen via both Fab arms, if two antigens are located close to each other on the biosensor surface so that the antibody can bind to both of them. This heterogenous mixture of antibodies bound via one Fab arm and via two Fab arms leads to complex signals which are difficult to analyze.

Hence, there is a need for a system which enables an exact control of the distance between antigens and therefore an exact analysis of the binding behavior of complex molecules such as bivalent and bispecific antibodies.

US 2003/0219803 discloses a method wherein an antibody is conjugated to an oligonucleotide and then contacted with a sample which may contain a target molecule of interest. Afterwards, the presence of the target molecule is detected by a set of cascade hybridization nucleic acids which may be fluorescently labeled.

**SUMMARY OF THE INVENTION**

The object of the present invention is solved by the subject-matter of the independent claims. Further embodiments and advantages of the invention are incorporated in the dependent claims.

The present invention provides a combination product which enables the exact spacing between two molecules and thereby an accurate analysis of the interaction between ligands and their binding partner, in particular the interaction of antigens with multivalent or multispecific antibodies such as bivalent or bispecific antibodies.

Hence, in a first embodiment the present invention provides a combination product comprising:

   (a) a nucleic acid structure having a sequence selected such that it forms at least one double strand (4) and at least one single-stranded loop (1); and

   (b)

      (i) one single-stranded nucleic acid molecule (6) which is capable of hybridizing to and forming a double strand with the single-stranded loop (1) and to which a first ligand (7) is attached to the 5' end of the single-stranded nucleic acid molecule (6) and a second ligand (8) is attached to the 3' end of the single-stranded nucleic acid molecule (6); or

(ii) a first (11) and a second (12) single-stranded nucleic acid molecule, wherein the first and the second nucleic acid molecules (11,12) are capable of hybridizing to and forming a double strand with different parts of the single-stranded loop (1) and wherein a first ligand (7) is attached to the first nucleic acid molecule (11) and a second ligand (8) is attached to the second nucleic acid molecule (12), wherein the at least one ligand (7,8) is a protein or a part thereof

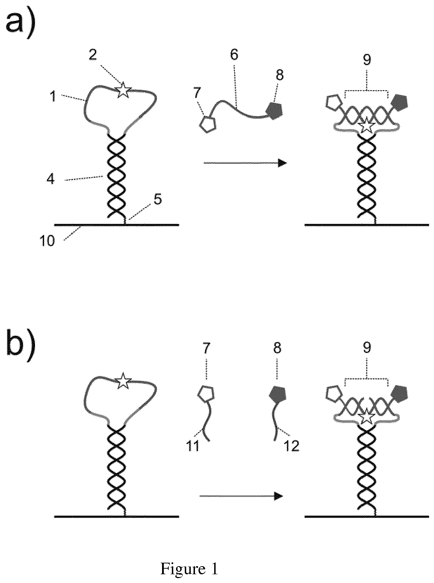

An exemplary combination product of this embodiment is illustrated in Figure 1.

Preferably, the nucleic acid structure is a stem-loop structure.

In a second embodiment the present invention provides a combination product comprising:

(a) a nucleic acid structure formed by three separate single-stranded nucleic acid molecules, wherein the first (13) and the second (14) single-stranded nucleic acid molecule each form a double strand with a first and a second part of the third single-stranded nucleic acid molecule (15) and wherein a third part of the third single-stranded nucleic acid molecule which is located between the first and the second part forms a single-stranded loop; and

(b) at least one single-stranded nucleic acid molecule (6) which is capable of hybridizing to and forming a double strand with the single-stranded loop and to which at least one ligand (7,8) is attached, wherein the at least one ligand (7,8) is a protein or a part thereof.

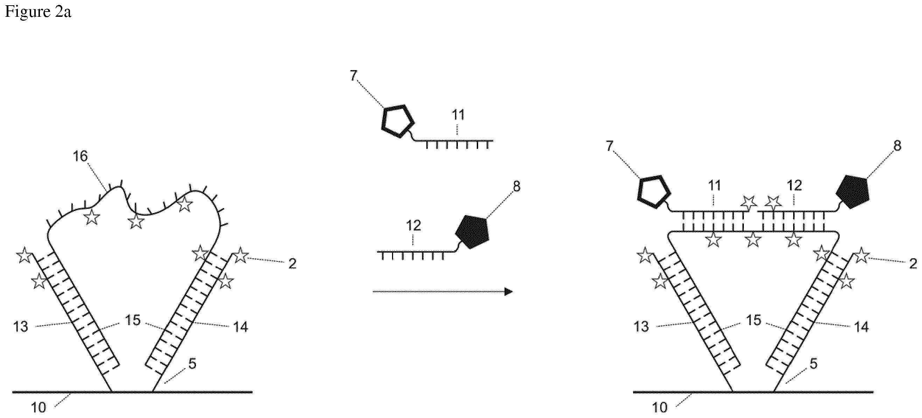

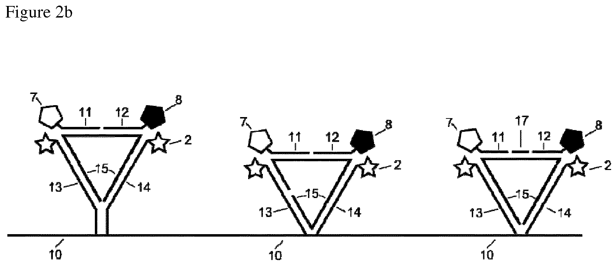

An exemplary combination product according to this embodiment is illustrated in Figure 2.

The combination product of the second embodiment of the present invention may comprise one single-stranded nucleic acid molecule (6) which is capable of hybridizing to and forming a double strand with the single-stranded loop and to which a first ligand (7) is attached to the 5' end of (6) and a second ligand (8) is attached to the 3' end of (6).

Alternatively, the combination product of the second embodiment of the present invention may comprise a first (11) and a second (12) single-stranded nucleic acid molecule, wherein the first and the second nucleic acid molecules (11,12) are capable of hybridizing to and forming a double strand with different parts of the single-stranded loop (1) and wherein a first ligand (7) is attached to the first nucleic acid molecule (11) and a second ligand (8) is attached to the second nucleic acid molecule (12).

The first ligand (7) and the second ligand (8) may be the same or different.

Also preferably, the nucleic acid structure is attached to a substrate (10).

In a further preferred embodiment the nucleic acid structure is labeled with a fluorophor (2).

The present invention also provides a substrate to which the combination product of the present invention is attached and a kit comprising the combination product or the substrate and instructions for its use.

The combination product or the substrate of the present invention may be used for the detection and/or characterization of the interaction of a ligand with at least one binding partner thereof, for protein purification or for the diagnosis of diseases.

In another aspect, the present invention provides a method for detecting and/or characterizing molecular interactions comprising:

(i) providing a combination product of the first embodiment of the present invention, wherein the single-stranded nucleic acid molecule (6) or the first and the second single-stranded nucleic acid molecule (11,12) forms a double-strand with the single-stranded loop; or
providing a combination product of the second embodiment of the present invention, wherein the at least one single-stranded nucleic acid molecule (6, 11, 12) forms a double-strand with the single-stranded loop;
(ii) contacting the combination product with a sample of the binding partner which is to be analyzed; and
(iii) detecting and/or characterizing the interaction between the ligand and the binding partner.

In still a further aspect, the present invention provides a method for detecting and/or characterizing molecular interactions comprising:

(i) contacting a single-stranded nucleic acid molecule (6) to which a first ligand (7) is attached to the 5' end of the single-stranded nucleic acid molecule (6) and a second ligand (8) is attached to the 3' end of the single-stranded nucleic acid molecule (6) or contacting a first (11) and a second (12) single-stranded nucleic acid molecule, wherein a first ligand (7) is attached to the first nucleic acid molecule (11) and a second ligand (8) is attached to the second nucleic acid molecule (12), with a sample of the binding partner which is to be analyzed, thereby providing a first mixture;
(ii) contacting the first mixture with the nucleic acid structure of the first embodiment and allowing the single-stranded

4

nucleic acid molecule to form a double-strand with the single-stranded loop; and

(iii) detecting and/or characterizing the interaction between the ligand and the binding partner.

In still a further aspect, the present invention provides a method for detecting and/or characterizing molecular interactions comprising:

(i) contacting at least one single-stranded nucleic acid molecule (6, 11, 12) to which at least one ligand (7,8) is attached with a sample of the binding partner which is to be analyzed, thereby providing a first mixture;
(ii) contacting the first mixture with the nucleic acid structure of the second embodiment and allowing the single-stranded nucleic acid molecule to form a double-strand with the single-stranded loop; and
(iii) detecting and/or characterizing the interaction between the ligand and the binding partner.

Preferably, the ligand is an antigen and the binding partner is an antibody, more preferably the antibody is a bispecific antibody.

In a preferred embodiment of the above methods, the combination product or the nucleic acid structure is attached to a substrate and is labeled with a fluorophor and the interaction is detected and/or characterized by the steps of:

(iv) applying an electrical field to the substrate; and
(v) detecting one of emission and quenching of fluorescence of said fluorophor, wherein a difference in the fluorescence upon contact with the sample indicates an interaction of the binding partner with the at least one ligand.

In a further preferred embodiment of the above methods, the combination product is attached to a substrate, the substrate has an electrode, the combination product is labeled with a fluorescent dye and the interaction is detected and/or characterized by the steps of:

(iv) applying an external electrical field to the electrode to cause a movement of said combination product;
(v) observing a signal of the label during said movement of the combination product, and
(vi) using the observed signal for detecting the interaction of the binding partner with the at least one ligand.

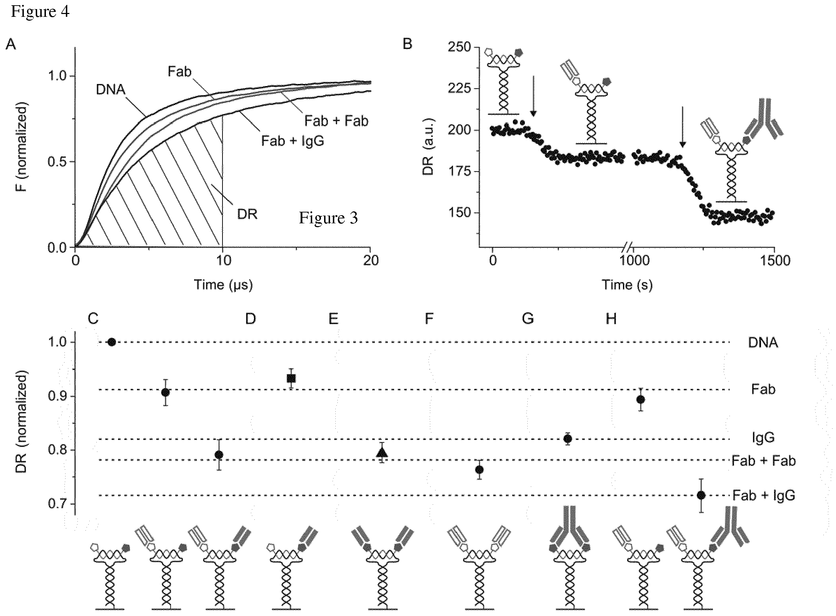

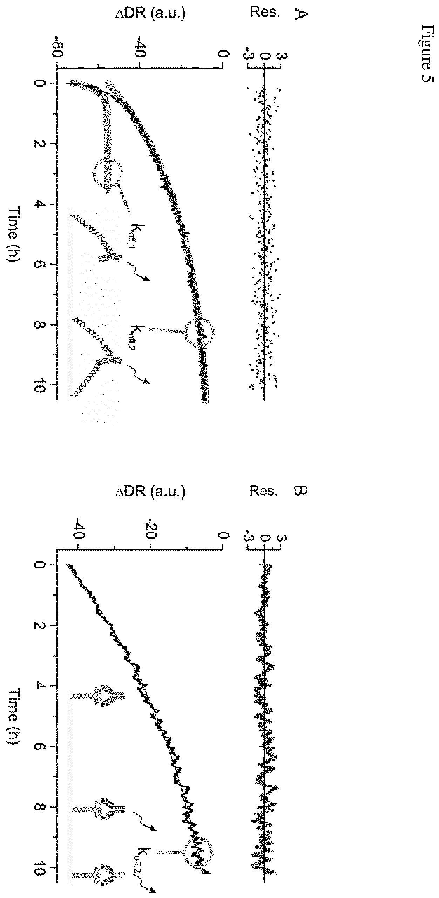

In other words, a method for detecting the interaction of the binding partner with the at least one ligand with oscillating molecules is presented. In this context, the oscillating molecules shall be understood as the combination product which comprises the nucleic acid structure and the at least one single-stranded nucleic acid molecule which has hybridized to the nucleic acid structure. Thus, the oscillating molecules are the molecules of the moved combination product. Said combination product can be switched by means of the external electrical field from a lying state into a standing state and vice-versa. This measurement principle can be gathered from, for example, the scientific publication "Detection and Size Analysis of Proteins with Switchable DNA Layers", Nano Letters, 2009, Vol. 9, 1290 - 1295. By causing the movement of the combination product from the lying into the standing position, a dynamical method for detecting binding interactions is provided. This method facilitates the detection based on a change of the dynamical behaviour of the combination product due to the binding of a binding partner to a ligand of the combination product. These aspects of the present invention make use of the fact that the combination product is electrically charged and thus has a polarity. More details hereof will be explained in the context of the Examples and Figures hereinafter. In other words, in this embodiment the herein presented method makes use of the "Switchsense" technology as has been described before. In further embodiments of this method the detection is based on an observed change of a hydrodynamic resistance of the switched molecules. Further details thereof will be explained in the context of the Figures, particularly in the context of Figures 4 and 5.

According to another exemplary embodiment of the invention quenching the signal of the label is used, wherein the quenching mechanism used depends on the distance between the quenching medium and the label. Thus, the used quenching is distant dependent.

For example, on a biochip a quenching layer, which is used also as an electrode, may be provided to facilitate the quenching of electromagnetic radiation emission from the label. An applied bias polarizes the electrode, leading to the formation of a Gouy-Chapman-Stern screening layer. Non-radiative energy transfer from the label to surface plasmons in the quenching layer may quench the emitted signal intensity when the label approaches the surface. Therefore, high signal intensities indicate a standing combination product configuration and less quenching during negative applied electrode bias. Low signal intensities indicate a lying combination product configuration during positive applied electrode bias. The latter is the case for a quenching layer being close to the substrate. However, said quenching layer may also be positioned opposite of the electrode, e.g. on the counter electrode.

If the electrode is a metal, a photoluminescent (PL) label which emits low photoluminescence, even when irradiated with light of an absorbable wave length as long as it interacts with the metal, for example when positioned near the metal, but which is capable of emitting photoluminescence in response to the light energy when exposed to light at an absorbable wave length, if it does not interact with the metal, for example when positioned away from the metal, is particularly suitable for using the emitting/quenching mechanism described herein. The label may be selected according to the following aspects. The absorption spectrum of the quenching medium coincides to some extent with the emission spectrum of the label, such that non-radiative energy transfer is possible. For example, the label may be a PL emitter and can be an organic dye molecule or also a nanoparticle.

The present invention also related to a method for separating a target protein from impurities comprising the steps of:

(a) providing a substrate of the present invention, wherein the at least one single-stranded nucleic acid molecule (6, 11, 12) forms a double-strand with the single-stranded loop;
(b) incubating the substrate with a sample containing the target protein under conditions allowing the target protein to bind to the ligand;
(c) washing the substrate under suitable conditions to remove impurities; and
(d) releasing the purified target protein from the substrate.

## BRIEF DESCRIPTION OF THE DRAWINGS

Figure 1: Schematic drawing of a first representative combination product of the present invention

a) The nucleic acid structure (also called slingshot structure) consists of a double strand (4, also called handle) and a single-stranded loop (1; also called sling) which is labeled with a fluorescent dye (2). One of the nucleic acid strands forming the double strand (4) is attached to a substrate (10) by a linker (5). Upon contact with a single-stranded nucleic acid molecule (6, also called c-spacer) to which a first and a second ligand (7,8) is attached a double strand (9, also called spacer bar) forms between the single-stranded loop and the single-stranded nucleic acid molecule.

b) The nucleic acid structure is the same as in a). Each of the two separate single-stranded nucleic acid molecules (11, 12) to each of which a ligand (7, 8) is attached forms a double strand with the single-stranded loop.

Figure 2: Schematic drawing of a second representative combination product of the present invention

a) Two single-stranded nucleic acid molecules (13, 14, also called handle 1 and handle 2) are each attached to a substrate (10) by a linker (5). A third single-stranded nucleic acid molecule (15; also called connector) forms a double strand with both single-stranded nucleic acid molecules (13, 14) and a single-stranded loop (16), thereby creating a structure called "triangular arch". Each of the two separate single-stranded nucleic acid molecules (11, 12; also called c-spacers) to each of which a ligand (7, 8) is attached forms a double strand with the single-stranded loop.
b) Variations of the second representative combination product of the present invention
The numbering of the different elements is the same as in a). In the variant on the right side, a small single-stranded nucleic acid molecule without a ligand (17) is hybridized to a part of the single-stranded loop in addition to the c-spacers.

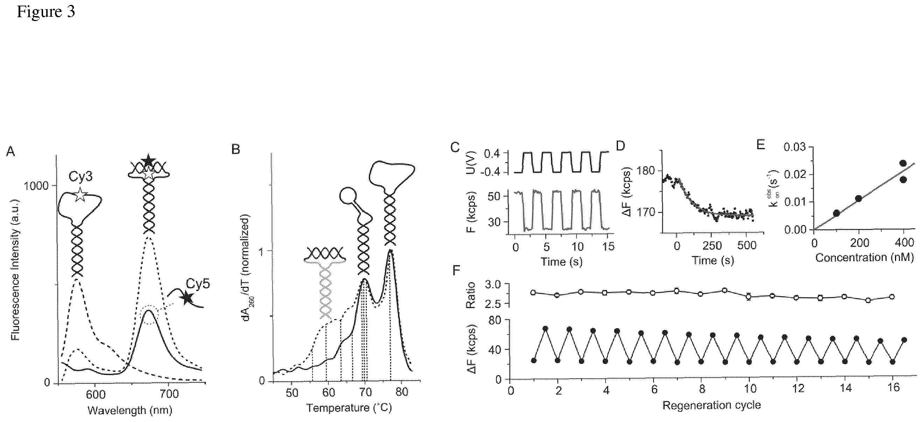

Figure 3: Formation of double strands by hybridization of a single-stranded nucleic acid molecule to the single-stranded loop of the nucleic acid structure

a) Hybridization of complementary spacer strand (c-spacer) to the sling in solution monitored by FRET. The central nucleotides of sling and c-spacer were labelled with Cy3 (green, donor) and Cy5 (red, acceptor) dyes, respectively. Long dashed and short dashed lines are spectra of individual slingshot and c-spacer sequences in solution, the solid line is a mixture ($c_{slingshot}$=90nM, $c_{c\text{-spacer}}$=100nM, $\lambda_{exc}$=530 nm).

b) Melting analysis of slingshot sequences with (dashed line) and without (continuous line) added c-spacer by UV hyperchromism in solution ($c_{DNA}$=1$\mu$M). Dashed vertical lines are calculated melting temperatures (mfold).

c) Electrical orientation switching of the nucleic acid structures on a gold surface. The Cy3 fluorescence F is high for standing and low for lying orientations, respectively.

d) Real-time measurement of the hybridization of c-spacer sequences (200 nM) to slingshot structures immobilized on gold surfaces. ΔF is the fluorescence modulation amplitude of the electrically actuated slingshot structure. The solid line is a single exponential fit, from which the rate constant *konobs* of hybridization is analyzed.

e) $k_{on}^{obs}$ as a function of c-spacer concentration in solution.

f) Regeneration: repeated removal of c-spacer sequence from immobilized slingshot structures by pH13 denaturation and re-hybridization with fresh c-spacer.

Figure 4: Binding of full-length antibodies and antibody fragments to the combination product wherein either two identical or two different antigens are attached to the c-spacer molecule.

a) Time-resolved fluorescence signal during the upward switching of the slingshot driven at 10kHz/±0.4V. The Dynamic Response parameter (DR, area under the time-resolved fluorescence trace) is a number representing the switching velocity; high DR values denotes fast switching.

b) Exemplary real-time binding response of Fabs and IgGs binding bispecific (dig/bio) combination products.

c) Equilibrium binding signals of combination products loaded with different combinations of Fab fragments and full IgGs. Open symbols denote dig/antidig, full symbols are biotin/antibiotin. DR values are normalized by the DR of combination products not bound by antibody.

Figure 5: Analysis of dissociation kinetics

a) Dissociation of anti-biotin IgG from a monovalent antigen probe or two monovalent antigen probes. The thin continuous line is a double-exponential fit, thick gray lines are plots of the two individual exponential components.

b) Dissociation of anti-biotin IgG from the combination product of the present invention. The continuous line is a single exponential fit.

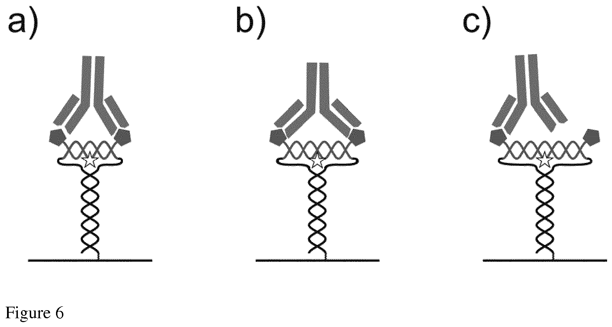

Figure 6: Analysis of possible binding conformations by varying the length of the single-stranded nucleic acid molecule.

a) At a length of about 20 nucleotides (corresponding to 6 nm) the bivalent binding of the antibody to both ligands of the c-spacer is possible.

b) At a length of about 30 nucleotides (corresponding to 9 nm) the bivalent binding of the antibody to both ligands of the combination product is still possible, but stressed due to a stretched conformation and a lower binding energy.

c) At a length of about 40 nucleotides (corresponding to 12 nm) the bivalent binding of the antibody to both ligands of the combination product is not possible.

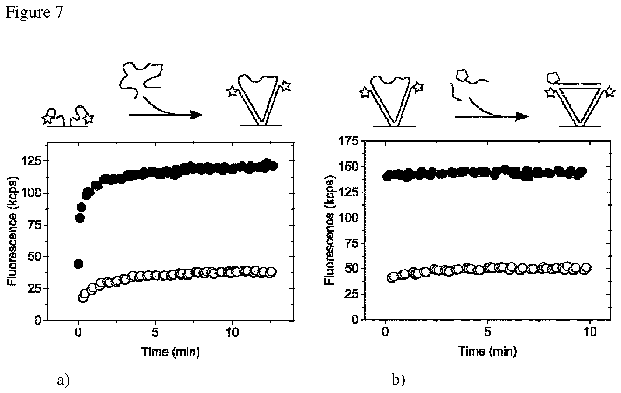

Figure 7: Formation of the "triangular arch" structure (see Figure 2) and hybridization of the structure with a single-stranded c-spacer

a) Hybridization of the connector strand to the surface-immobilized handles 1 and 2 followed in real-time by fluorescence measurements. The open symbols show the fluorescence intensity at +0.4 V and the filled symbols show the fluorescence intensity at -0.4 V.

b) Hybridization of the single-stranded c-spacer to the single-stranded loop formed by the connector strand followed in real-time by fluorescence measurements. The open symbols show the fluorescence intensity at +0.4 V and the filled symbols show the fluorescence intensity at -0.4 V.

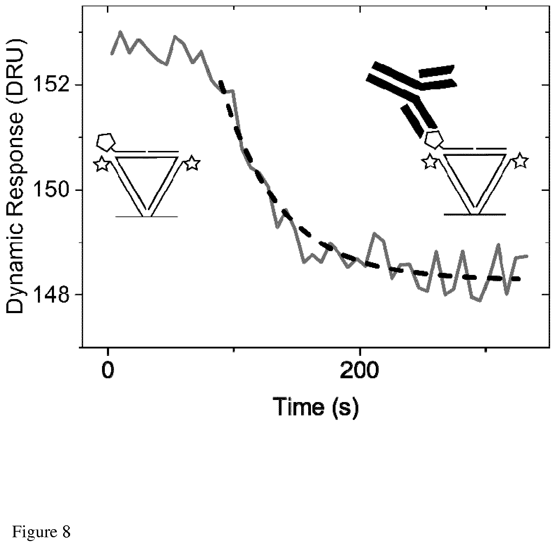

Figure 8: Binding of a full-length anti-biotin antibody to the "trinagular arch" structure (see Figure 2).

Time-resolved fluorescence signal during the upward switching of the slingshot driven at 10kHz/±0.4V. The Dynamic Response parameter (DR, area under the time-resolved fluorescence trace) is a number representing the switching velocity; high DR values denotes fast switching.

## DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto, but only by the claims.

Where the term "comprising" is used in the present description and claims, it does not exclude other elements. For the purposes of the present invention, the term "consisting of' is considered to be a preferred embodiment of the term "comprising". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm20$ %, preferably $\pm15$ %, more preferably $\pm10$ %, and even more preferably $\pm5$ %.

Furthermore, the terms "first", "second", "third" or "(a)", "(b)", "(c)", "(d)" or "(i)", "(ii)", "(iii)", "(iv)" etc. and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps unless indicated otherwise, i.e. the steps may be carried out simultaneously or there may be time intervals of seconds, minutes, hours, days, weeks, months or even years between such steps, unless otherwise indicated in the application as set forth herein above or below.

Technical terms are used by their common sense. If a specific meaning is conveyed to certain terms, definitions of terms will be given in the following in the context of which the terms are used.

The term "combination product" is intended to mean that the product contains at least two components, i.e. a nucleic acid structure and a single-stranded nucleic acid molecule which in the combination product is either hybridized to the nucleic acid structure by forming a double strand with the single-stranded loop or remains separate.

Within the meaning of the present invention a "nucleic acid structure" is a secondary structure formed by one or more nucleic acid molecules. In a first embodiment (also called "slingshot") which is illustrated in Figure 1 it is formed by one nucleic acid molecule having a sequence selected such that it forms at least one double strand and at least one single-stranded loop, for example a stem-loop structure. In a second embodiment (also called "triangular arch") which is illustrated in Figure 2 the nucleic acid structure is formed by three separate single-stranded nucleic acid molecules, wherein the first and the second single-stranded nucleic acid molecule each forms a double strand with a first and a second part of the third single-stranded nucleic acid molecule and wherein a third part of the third single-stranded nucleic acid molecule which is located between the first and the second part forms a single-stranded loop. Unless explicitly stated otherwise the following discussion applies to both the first and the second embodiment of the nucleic acid structure.

The term "nucleic acid" refers to any type of nucleic acid molecule such as DNA or RNA or PNA or LNA with DNA being the preferred nucleic acid.

Within the meaning of the present invention the term "hybridizes to" is intended to mean that the single-stranded nucleic acid molecule forms a double strand with the single-stranded loop of the nucleic acid structure by the interaction of complementary bases in the single strands.

The person skilled in the art knows how to select a sequence such that it forms a double strand and a single-stranded loop. In particular, it is well-known that a nucleotide double-strand forms by forming hydrogen bonds between complementary bases, i.e. between adenine and thymine or between cytosine and guanine. Hence, in the first embodiment of the present invention the molecule which forms a double strand has to contain a first sequence and a second sequence complementary to the first sequence. Similarly, in the second embodiment of the present invention the first single-stranded nucleic acid molecule has to be complementary to the first part of the third single-stranded nucleic acid molecule and the second single-stranded nucleic acid molecule has to be complementary to the second part of the third single-stranded nucleic acid molecule. The double strand forms if the two sequences are contacted under suitable conditions, i.e. a pH above 2, a salt concentration equivalent to more than 20mM NaCl and a temperature below the melting temperature of the double strand which is dependent on the length of the double strand. Suitable conditions for forming a double strand are also known to the skilled person and include the use of a buffer comprising 40 mM NaCl, 10 mM sodium phosphate buffer, pH 7.4 and 0.05% Tween 20 at a temperature of 25°C..

The sequences forming the double strand do not have to be 100% complementary to each other, meaning that in the double strand one or more bases may not be paired with their complementary base. The person skilled in the art knows that longer double-stranded molecules permit a larger number of non-pairing bases than shorter double-stranded molecules without significantly influencing the stability of the double strand. In general, at least 50%, preferably at least 60% and more preferably at least 70% of the bases within the sequences forming the double strand should be complementary to each other.

For the sequence to form a single-stranded loop it is necessary that the sequence within the loop does not show significant complementarity to the other sequences within the nucleic acid structure so that no double-stranded structures are formed between the sequence within the loop and the first or second sequence of the double strand.

In the nucleic acid structure additional nucleotides may be present between the sequences forming the double strand and the sequence forming the single-stranded loop which nucleotides are not part of the double strand of the nucleic acid structure and which also do not take part in the formation of the double strand between the single-stranded loop and the single-stranded nucleic acid molecule. In a particular embodiment, the sequence connecting the double strand(s) with the single-stranded loop is a sequence consisting of only one kind of nucleotide, preferably the sequence consists exclusively of thymidine nucleotides. The insertion of this connecting sequence may help to avoid base-stacking interactions and to allow for flexibility. The length of the sequence connecting the double strand(s) with the single-stranded loop depends on the length of the double strand formed by the single-stranded loop and the single-stranded nucleic acid molecule and may be up to 50% of the length of this double strand.

If the nucleic acid structure is to be attached to a substrate, one or both of the sequences forming the double strand may be attached to a linker which facilitates the attachment to the surface. Suitable linker molecules are known to the skilled person and depend on the surface to which the nucleic acid structure is to be attached. If the substrate is a gold surface, the linker is preferably a thiol linker such as $(CH_2)_6$-SH or di-thiol phosphoramidite. For glass or silicium surfaces a silane linker may be used. If the surface is modified with avidin, the linker may be biotin. An overview of further linkers is provided on the website of the company Integrated DNA Technologies (IDT). Methods for attaching a linker to a nucleic acid molecule are well-known and include the use of thiolated nucleotides (see Hegner et al. (1993) FEBS 336(3): 452-456). Nucleic acid molecules with an attached linker can be obtained commercially from companies such as Eurofins Genomics or atdbio.

In a preferred embodiment the nucleic acid structure is a stem-loop structure that has one single-stranded loop which is associated with one double-stranded stem. Such structures are well-known to the skilled person.

The double strand within the nucleic acid structure may have a length of 5 to 300, preferably 10 to 200 or 15 to 150, more preferably 18 to 120 or 20 to 100, even more preferably 23 to 80 or 25 to 60 and most preferably 30 to 50 base pairs.

The double strand may consist of DNA, RNA, PNA and LNA homoduplexes and heteroduplexes thereof such as DNA/RNA, DNA/PNA, DNA/LNA, RNA/PNA and PNA/LNA. The skilled person knows that the kind of nucleic acid used may influence the stability of the double strand so that the minimal length required for the formation of a stable double strand depends on the type of nucleic acid forming the double strand.

The single-stranded loop may have a length of 10 to 170, preferably of 11 to 150 or 12 to 120, more preferably of 13 to 120 or 14 to 100, even more preferably of 15 to 90 or 16 to 70, particularly preferably of 17 to 50 or 18 to 40 and most preferably of 20 to 30 nucleotides. Similar to the double strand the single-stranded loop may consist of DNA, RNA, PNA and LNA.

The at least one single-stranded nucleic acid molecule which is capable of forming a double strand with the single-stranded loop may have a length of 10 to 170, preferably of 11 to 150 or 12 to 120, more preferably of 13 to 120 or 14 to 100, even more preferably of 15 to 90 or 16 to 70, particularly preferably of 17 to 50 or 18 to 40 and most preferably of 20 to 30 nucleotides. The single-stranded nucleic acid molecule may consist of DNA, RNA, PNA and LNA. It is not required that the single-stranded nucleic acid consists of the same type of nucleic acid as the double strand or the single-stranded loop.

To be able to hybridize to the single-stranded loop the sequence of the single-stranded nucleic acid molecule(s) has to be complementary to the sequence of the single-stranded loop. The sequences forming the double strand do not have to be 100% complementary to each other, meaning that in the double strand one or more bases may not be paired with their complementary base. The person skilled in the art knows that longer double-stranded molecules permit a larger number of non-pairing bases than shorter double-stranded molecules without significantly influencing the stability of the double strand. In general, at least 50%, preferably at least 60% and more preferably at least 70% of the bases within the sequences forming the double strand should be complementary to each other.

It is not necessary for the single-stranded nucleic acid molecule to have the same length as the single-stranded loop, as long as the single-stranded nucleic acid molecule is able to form a stable double strand with the single-stranded loop by hybridizing to each other. Hence, the single-stranded loop may comprise nucleotides which do not form part of the double strand with the single-stranded nucleic acid molecule. Alternatively, the single-stranded nucleic acid molecule may comprise nucleotides which do not form part of the double strand with the single-stranded nucleic acid loop. Further, the single-stranded nucleic acid molecule does not comprise nucleotides capable of forming a double strand with the double strand (4) of the nucleic acid structure of the first embodiment or with the double strand formed between the first (13) and the second (14) single-stranded nucleic acid molecule and the first and second part of the third nucleic acid molecule in the nucleic acid structure of the second embodiment.

Upon formation of the double strand between the single-stranded nucleic acid molecule(s) and the single-stranded loop the double strand (4) within the nucleic acid structure of the first embodiment is not disrupted, i.e. it remains intact. Similarly, in the nucleic acid structure of the second embodiment the double strand formed between the first (13) and the second (14) single-stranded nucleic acid molecule and the first and second part of the third nucleic acid molecule is not disrupted, i.e. it remains intact, upon formation of the double strand between the single-stranded nucleic acid molecule(s) and the single-stranded loop.

In one embodiment the combination product comprises one, i.e. a single, single-stranded nucleic acid molecule which is capable of hybridizing to and forming a double strand with the single-stranded loop. To each end of the single-stranded nucleic acid molecule, i.e. the 5' and the 3' end, a ligand is attached.

The length of the single-stranded nucleic acid molecule determines the distance between the two ligands attached to it. Hence, it is possible to vary the distance between the two ligands by varying the length of the single-stranded nucleic acid which enables the analysis of spacing between binding sites in the molecule interacting with the ligand and of the influence of the distance on the binding energy.

In an alternative embodiment the combination product comprises two separate single-stranded nucleic acid molecules, each of which is attached to one ligand. The two single-stranded nucleic acid molecules are capable of hybridizing to separate, non-overlapping parts of the single-stranded loop within the nucleic acid structure. It is not necessary that the two nucleic acid molecules together bind over the whole length of the single-stranded loop. It is also not necessary that the two nucleic acid molecules bind adjacent to each other, meaning that a part of the single-stranded loop may not be bound by any of the single-stranded nucleic acid molecules to form a double strand, but remains single-stranded. Further, it is not necessary for the two single-stranded nucleic acid molecules to have the same length, as long as both are able to form a stable double strand with the single-stranded loop.

In this embodiment, the length of the single-stranded loop sequence to which both single-stranded nucleic acid molecules hybridize determines the distance between the ligands attached to the separate nucleic acid molecules. If the two single-stranded nucleic acid molecules hybridize to adjacent parts of the single-stranded loop, the distance between the two ligands attached to the single-stranded nucleic acid molecules is the sum of the length of these two single-stranded nucleic acid molecules. If the two single-stranded nucleic acid molecules do not hybridize to adjacent parts of the single-stranded loop, a third single-stranded molecule may be hybridized to those nucleotides in the single-stranded loop which are located between the nucleotides to which the two single-stranded nucleic acid molecules hybridize. This third single-stranded nucleic acid molecule allows to modulate the distance between the ligands attached to the two single-stranded nucleic acid molecules without having to modulate these molecules themselves.

The person skilled in the art is aware of methods for attaching ligands such as proteins to nucleic acid molecules. One possibility is to modify the nucleic acid molecule by 6-maleimidohexanoic acid *N*-hydroxysuccinimide ester and then to react the modified DNA with the protein having suitable amino acid residues for conjugation (see, e.g., Fujiwara et al. (1988). J. Immunol. Methods 112: 77-83; Peeters et al. (1989). J. Immunol. Methods 120: 133-143). Further, kits are available for producing protein-nucleic acid conjugates, for example from Solulink. An overview of different conjugation techniques is provided in Hermanson, Bioconjugate Techniques, Elsevier, third edition 2013 and in Mark, Bioconjugation Protocols, Humana Press, second edition 2011.

A "ligand" is a molecule which can bind to another molecule by non-covalent bonds due to a specific affinity to the other molecule. Within the meaning of the present invention the ligand is a protein or part thereof. The part of the protein may have a minimum length of at least six amino acids which is the minimum length of an epitope which is recognized by an antibody.

The first and the second ligand attached to the single-stranded nucleic acid molecule may be the same, i.e. they may have the same chemical structure. For example, if the interaction of a bivalent, monospecific full-length antibody with an antigen is to be analyzed, the first and the second ligand may be this antigen.

In an alternative embodiment, the first and the second ligand attached to the single-stranded nucleic acid molecule may be different, i.e. they may have a different chemical structure. In the case of proteins, different proteins or parts of different proteins may be used as first and second ligand. For example, if the interaction of a bispecific antibody with its target antigens is to be analyzed, the first ligand may be the first antigen or part thereof and the second ligand may be the second antigen or part thereof, wherein the first and the second antigens are the target antigens of the bispecific antibody. Alternatively, different parts of the same protein may be used as first and second ligand which for example allows determining the binding epitope of an antibody. In a further alternative, the first and the second ligand may have the same amino acid sequence, but may differ in post-translational modifications such as phosphorylations, methylations and acetylations. This embodiment allows the analysis of the influence of post-translational modifications on the binding of a binding partner such as the binding of an antibody to its target antigen.

If the binding partner to be analyzed with the combination product of the present invention is a signal transduction molecule, the first ligand may be a protein which is supposed to be upstream of the binding partner in the signal transduction cascade and the second ligand may be a protein which is supposed to be downstream of the binding partner in the signal transduction cascade.

The nucleic acid structure part of the present combination product may be attached to a substrate. The term "substrate" is to be understood in its broadest sense as a structure to which a nucleic acid molecule can be coupled reversibly or irreversibly and includes, for example, planar matrices such as chips or beads. The substrate may be made of any substance, like e.g. glass, to which a nucleic acid molecule can be attached and may be made, e.g. from gold or other metals. For switching applications as described herein, a biochip may be used which comprises a film on top of said substrate. For example, a gold film of 5-300 nm thickness may be used on a glass substrate or an organic layer, e.g. a conducting polymer or a dye-sensitized matrix may be used on the substrate. This layer may function as a quenching layer used in an embodiment of the detection method.

As discussed above, to attach a nucleic acid molecule to a substrate a linker molecule such as a thiol may be required which is attached to the nucleic acid structure and enables the binding of the nucleic acid structure to the substrate by a chemical reaction. According to another exemplary embodiment of the invention the chemical linker is chosen from the group that contains one of-or a combination of- the following reactive groups: aldehyde, ketone, thiol, amine, carboxyl, hydrazine, hydrazide, hydroxyl, glycan, azide, alkyne, alkene, silicon, and any combination thereof.

In a preferred embodiment of the present invention the substrate is a gold surface to which the nucleic acid structure of the combination product is coupled by means of a thiol linker which is attached to and or all of the nucleic acid molecules forming the double strand.

The present invention also relates to a substrate to which at least one combination product of the present invention is attached via the nucleic acid structure. To the substrate two, three, four, five, six, seven, eight, nine or ten, 12, 14, 16, 18, 20, 22, 24, 26, 28 or 30 combination products may be attached. Typically, but not necessarily, the nucleic acid structure and the single-stranded nucleic acid molecule are the same in all combination products attached to the substrate and the combination products will only differ in the first and/or the second ligand attached to the single-stranded nucleic acid molecule. If two separate single-stranded nucleic acid molecules are used in the combination product, it is possible to use the same first ligand in all combination products attached to the substrate and to vary only the second ligand within the substrate. In this way, many different molecule interactions may be analyzed in parallel by varying only one component of the combination product.

Further, after analyzing one or more interactions on a substrate with a specific single-stranded nucleic acid molecule or a set of different specific single-stranded nucleic acid molecules, the substrate with the nucleic acid structure attached to it can be used for the analysis of further interactions after removing the single-stranded nucleic acid molecule from the nucleic acid structure. The single-stranded nucleic acid molecule can for example be removed by treatment with a strongly basic solution such as a solution with pH 13 which denatures the double strand between the single-stranded loop and the single-stranded nucleic acid molecule. After removing the first single-stranded nucleic acid molecule or set of single-stranded nucleic acid molecules from the nucleic acid structure, the single-stranded loop can be hybridized with single-stranded nucleic acid molecules having other ligands attached to them. The present inventors have shown that the nucleic acid structure can be re-used at least 16 times without significantly influencing the quality of the analysis.

If the combination product of the present invention is to be used for example in the "Switchsense" method, the nucleic acid structure may be labeled with a fluorescent dye. Suitable fluorescent dyes are known to the skilled person and include FITC, fluoresceine, rhodamine green, rhodamine red, cyanine3 and cyanine5. Preferably, cyanine 3 and/or cyanine5 are used. Nucleic acid molecules which are labeled with fluorescent dyes can be obtained from different suppliers such as Eurofins Genomics or Genscript. Within the nucleic acid structure forming a double strand and a single-stranded loop the fluorescent dye may be located both in the single-stranded loop and in the double strand. Within the nucleic acid structure formed by three separate nucleic acid molecules the fluorescent dye may be located in the first and/or second single-stranded nucleic acid molecule or in the single-stranded loop.

The combination product of the present invention can be used to detect and/or analyze interactions between molecules, in particular the interaction of one or more antigens with an antibody. The term "detecting" means that it can be determined whether such an interaction indeed exists or not. The characterization of an existing interaction includes the determination of parameters such as affinity and/or avidity of the molecules to each other, the binding kinetics, i.e. the kinetics of association and dissociation between two molecules, the size of the molecules, conformational changes in the three-dimensional structure of the molecules, chemical modifications changes in the charge of the molecules, binding energies (free energy, enthalpy, entropy) in equilibrium, energy barriers involved in association or dissociation processes and the like.

The combination product of the present invention can also be used in the purification of proteins, i.e. the separation of proteins from other proteins and impurities present in a sample. For example, the nucleic acid structure of the combination product may be attached to beads such as polystyrene or magnetic beads and the single-stranded loop forms a double strand with the single-stranded nucleic acid molecule. After incubation of the attached combination product with a sample containing the protein to be purified, the beads may be washed under suitable conditions to remove impurities and then the purified protein may be released from the beads.

Further, the combination product of the present invention can be used in the diagnosis of diseases in which two or more proteins bind to each other in a defined distance to form agglomerates. In this case, the combination product is construed to bind only to the proteins forming the agglomerates, but not to protein monomers or multimers in which the proteins have another distance to each other.

The present invention is described with particular reference to the analysis of antigen/antibody interactions by the so-called "switchSENSE" technology. However, it becomes apparent from the present description that the present invention can also be used to detect and/or analyze interactions of other molecules by other detection techniques such as surface plasmon resonance.

## EXAMPLES

### 1. Biochip

The biochip consists of a glass substrate (27 x 40 mm) with eight holes (1 mm diameter) which serve as in- and outlets for four flow channels. Au work electrodes (120 $\mu$m diameter) and Pt counter electrodes were arranged in 4 areas with 6 electrodes each and fabricated by standard optical lithography and metallization techniques. Prior to DNA immobilization, the surface was cleaned in freshly prepared Piranha solution (95%$H_2SO_4$:30%$H_2O_2$ = 2:1) for 15 minutes, followed by extensive rinsing with deionized water, 3 min sonication and drying with nitrogen.

### 2. Preparation of "standard" DNA layers

Thiolated 48mer oligonucleotides were end-grafted to the gold electrodes via spotting with a pico-liter dispensing system in immobilization buffer (10 mM Tris pH 7.4, 200 mM NaCl, 1 $\mu$M DNA). The sequence of the Cy3 labelled ss-DNA for immobilization was 5' HS-(CH$_2$)$_6$-TAG TCG TAA GCT GAT ATG GCT GAT TAG TCG GAA GCA TCG AAC GCT GAT-Cy3 3' (Metabion, Germany). After 10 min incubation, the chip was assembled by using double adhesive film with die-cut flow channels as an intermediate layer and a cover slide as a top layer. The flow channels were 60 $\mu$m high and 1 mm wide and covered one of four electrode areas each. The DNA-modified Au electrodes were passivated and unspecifically bound DNA was removed by co-adsorbing mercaptohexanol (ImM in 'T'-buffer: 10 mM Tris pH 7.4, 50 mM NaCl) for 30 min. After installation in the setup, the DNA layers were hybridized with complementary DNA and the density was adjusted using a previously reported electrical desorption procedure[40].

Briefly, the relative switching amplitude $\Delta$F/F ($\Delta$F is the observable fluorescence modulation amplitude when applying repulsive or attractive potentials, and F is fluorescence at repulsive potential) is used as a parameter that indicates the free mobility of DNA strands on the surface. Due to steric interactions, densely packed DNA levers cannot lie down on the surface, i.e. cannot be switched ($\Delta$F/F $\approx$ 0%), while ultra-low-density layers feature $\Delta$F/F values close to 100%. By applying negative voltage pulses (e.g. -0.8V vs. Pt) for a couple of seconds, DNA was electrically desorbed from the surface until $\Delta$F/F became maximal, indicating free DNA movement in a very dilute layer (density estimation < $10^{10}$ molecules/cm$^2$).

If required, double stranded DNA layers were denatured by flowing NaOH solution (pH 12-13) over the electrodes for 10s. The layers could then be regenerated by hybridization with fresh complementary DNA (50 nM in T-buffer). A flow rate of 100$\mu$l/min was typically used during sensing experiments, which was high enough to operate the sensor in the reaction-limited kinetics regime. An effect of the applied hydrodynamic flow on the DNA switching behaviour was not observed.

### 3. Preparation of DNA layers of the present invention (also called "Slingshot layers")

For the purpose of the following discussion the nucleic acid structure according to the first embodiment of the present invention is also called slingshot or slingshot structure. The double strand within this structure is also called handle and the single-stranded loop is also called sling. The single-stranded nucleic acid molecule is also called c-spacer and the double strand formed between the single-stranded loop and the single-stranded nucleic acid molecule is also called spacer bar.

Slingshot layers were prepared in the same way as standard layers. The slingshot sequences were: Handle and sling: (90 mer) 5' Thiol- TAG TCG CCC GCT GAT ATG GCT GAT TCG TCG TTT TTG CAG CTG GT*G TCG AGA CGG GTT TTT CGA CGA ATC AGC CAT ATC AGC GGG CGA CTA -3';

spacer bar: (20mer) 5'-CCC GTC TCG ACA CCA GCT GC -3'.

Antigen modified spacer bar 5' - antigen1- CCC GT*C TCG ACA CCA GCT* GC - antigen2 - 3'.

Three different versions were prepared:

(1) antigen 1=biotin, antigen2=digoxigenin;
(2) antigen 1=biotin, antigen2=biotin;
(3) antigen1=digoxigenin, antigen2=digoxigenin.

Asterisks mark the internal labelling of nucleotides with Cy3 or Cy5, respectively.

## 4. Preparation of DNA layers of the present invention (also called "Triangular arch layers")

For the purpose of the following discussion the nucleic acid structure according to the second embodiment of the present invention is also called triangular arch structure. The immobilized single-stranded nucleic acid molecules are also called handles and the single- stranded nucleic acid molecule hybridizing to the handles forming a double strand is also called connector strand. The single-stranded nucleic acid molecule is also called spacer or c-spacer and the double strand formed between the single-stranded loop and the single-stranded nucleic acid molecule is also called spacer bar.

Triangular arch layers were prepared in the same way as standard layers. To keep the thiols in close proximity during the immobilization process a DNA strand called "immobilization connector" is hybridized to the single-stranded handle 1 and handle 2 before immobilization. After immobilization and desorption process the immobilization connector was dehybridized from the triangular arch layers as described above. For functionalization a single-stranded DNA strand called "connector" was hybridized to the immobilized handle 1 and handle 2 using a buffer containing 40 mM NaCl, 10 mM sodium phosphate buffer, pH 7.4 and 0.05% Tween 20 at 25°C. The concentration of the connector strand and the antigen-modified spacers was 200 nM each. Further functionalization processes were identical to the preparation of the slingshot layer.
The sequences were:

Handle 1:5' T*AG TCG TAA GCT GAT ATG GCT GAT TAG TCG GAA GCA TCG AAC GCT GAT - Thiol 3'
Handle 2: 5' Thiol - GTG TGA ACC CTC CAA CAA AGG TAG CAT TTG CCA GCT CTC GTG ATG CAG* 3'
Immobilization connector: 5' CTG CAT CAC GAG AGC TGG CAA ATG CTA CCT TTG TTG GAG GGT TCA CAC ATC AGC GTT CGA TGC TTC CGA CTA ATC AGC CAT ATC AGC TTA CGA CTA
Connector: ATC AGC GTT CGA TGC TTC CGA CTA ATC AGC CAT ATC AGC TTA CGA CTA T GGA CCC TAA GA G ACT GGT ACG T CTG CAT CAC GAG AGC TGG CAA ATG CTA CCT TTG TTG GAG GGT TCA CAC 3'
Spacer 1:5' biotin - CGT ACC AGT C 3'
Spacer 2: 5' TC TTA GGG TCC 3'

Asterisks mark the labelling of nucleotides with Cy3

## 5. Measurement setup

Biosensing measurements were performed with the DRX 2400 switchSENSE analyser instrument from Dynamic Biosensors GmbH. Fluorescent light from the Cy3-labeled DNA nucleic acid structures was excited by a green LED (517 nm bandpass filtered) that was coupled into a 50x objective via a dichroic mirror. Emitted Cy3 fluorescence light (570 nm) was collected by the same objective, passed through the dichroic mirror and a long pass filter, and detected with a photomultiplier (Hamamatsu). AC square-wave signals from a frequency generator were used to actuate the DNA molecules and simultaneously trigger an event timer of a single photon counting unit (NanoHarp, PicoQuant, Germany) to record the photon arrival time with 32 ns resolution. A Peltier element integrated in the sample holder enabled temperature controlled experiments.

Before every time-resolved (TR) fluorescence measurement, the static fluorescence response to an applied voltage ramp was recorded. The switching potentials for TR measurements were chosen to be close to the two 'plateau regions' of the fluorescence response (indicating completely lying and completely standing DNA). The voltage amplitude was between 0.6 V and 1 V, the DC offset ($\approx 0.0$ V) was adjusted (app. $\pm 0.1$ V) according to the "potential of conformation transition", i.e., the inflection point in the measured voltage response curve. The frequency of the applied square wave potential was 10 kHz, which ensured that the DNA had enough time to stand up and lie down completely before reversing the voltage.

The Dynamic Response is calculated from the normalized fluorescence signal $F_{\text{norm}}$ as

$$DR^{t2}_{t1\,\text{up}} = \int_{t1}^{t2} F_{\text{norm}} \mathrm{d}t, \quad DR^{t2}_{t1\,\text{down}} = \int_{t1}^{t2} (1 - F_{\text{norm}}) \mathrm{d}t,$$

for the upward and the downward motions, respectively.

## 6. Results

a) Characterization of the "slingshot layers"

To confirm that c-spacer correctly hybridizes to the sling fluorescence resonance energy transfer (FRET) measurements were performed. To this end c-spacer was labeled at its central position with Cy5, which acts as an acceptor for non-radiative ET from optically excited Cy3 fluorophores. Solutions containing only Cy3-labelled slingshot structures or Cy5-labelled c-spacers feature the typical spectra of the respective fluorophores. By contrast, the mixture of both sequences exhibits enhanced (suppressed) emission of Cy5 (Cy3), as expected when Cy5 is held in close proximity to Cy3 by a double strand formed between the c-spacer and the sling. The FRET efficiency calculated from the measured spectra is 0.7, which corresponds to the maximal efficiency anticipated under these experimental conditions (J. Phys. Chem. 122 061103 (2005), Iqbal et al. (2008) Proc. Natl. Acad. Sci. USA 105(32): 11176-11181). This suggests a hybridization efficiency close to 100%, meaning that practically all slings are hybridized to the c-spacers.

To characterize the thermodynamic properties of the DNA nanostructure melting experiments in solution were performed. The denaturation from a double- to a single-stranded conformation was monitored by absorption measurements at 260 nm, using the hyperchromic effect. For the sling (black line in Figure 3B) two transitions with melting temperatures $Tm,1=77°C$ and $Tm,2=70°C$ were found. By comparison with thermodynamic calculations with mfold (Zuker (2003) Nucl. Acids Res. 31 (13): 3406-3415), $Tm,1$ can be assigned to the melting of the 30 bp handle with an open sling, while the broader transition around $Tm,2$ originates from the melting of the handle in combination with a partly folded sling. This is not surprising, as the calculations suggest at least three different possible secondary structures within the sling. Remarkably, although the handle-&-partly-folded-sling structure features a larger number of base-pairs, it is less stable than the handle -&-open-sling structure. This results from the entropic burden imposed by secondary structures within the sling, which reduce the degrees of freedom of the sling.

When the 20 nt c-spacer is hybridized to the sling (dashed line in Fig. 3B), a third, broad melting transition can be observed at $Tm,3\approx59°C$. A comparison of experimental data and calculations for 20bp, 18bp, and 16bp duplexes formed by the sling and the c-spacer suggests that $18 \pm 2$ bases are paired between the sling and the c-spacer. Most likely, strain in the (dT)5 joint segments of the sling prevents the outermost nucleotides of the c-spacer from base-pairing. Thus, the rigid length of the duplex formed by the sling and the c-spacer is $18\times0.34$ nm = 6.1 nm here.

Next the slingshot structure was characterized on a biosensor surface, employing the recently introduced switchSENSE method, which uses the electrical actuation of DNA nanolevers to detect proteins (Langer et al. (2013) Nature Commun. 4: 2099; Knezevic et al. (2012) J. Am. Chem. Soc. 134(37): 15225-15228) and nucleic acids in real-time (Rant et al. (2007) Proc. Natl. Acad. Sci. USA 104(44): 17364-17369). To test whether the structure is suitable for this measurement modality, the nucleic acid structures were immobilized on gold surfaces using thiol modified handles.

Figure 3C shows that the orientation of the nucleic acid structures can be modulated efficiently by applying $\pm0.4V$ to the supporting microelectrodes (vs. an ITO counter electrode). The orientation switching is monitored by measuring the fluorescence emitted by Cy3 dyes attached to the center of the single-stranded loop. Whenever the negatively charged nucleic acid structures are attracted by positive potentials and lie on the surface, the fluorescence is quenched by non-radiative energy transfer to the gold film (Kaiser and Rant (2010) J. Am. Chem. Soc. 132(23): 7935-45. Conversely, when the nucleic acid structures are repelled by negative potentials and stand upright, the fluorescence emission is high.

The hybridization of the c-spacer to surface-immobilized nucleic acid structures can be followed in real-time when incubating structures with open slings with solute c-spacers, see Figure 3D. As the c-spacer binds to the sling and forms the double strand, the fluorescence modulation amplitude $\Delta F$ decreases because the sling is pulled towards the handle, which reduces the vertical extension of the standing structure. Association rate constants $k_{on}^{obs}$ were analyzed by fitting single exponential functions $((t)\propto exp\{- k_{on}^{obs} \cdot t\})$ to kinetic data measured for different concentrations of the single-stranded nucleic acid molecule, and the association rate $k_{on}=(5.3\pm0.4)\times10^4 M^{-1}s^{-1}$ was determined from a linear fit in Figure 3E ($k_{on}^{obs}=c\cdot k_{on}+k_{off}$) . This value agrees well with other reports on oligonucleotide hybridization on surfaces (Tawa and Knoll (2004) Nucl. Acids Res. 32(8): 2372-2377; Gao et al. (2006) Nucl. Acids Res. 34(11): 3370-3377), but is lower than what is expected for hybridization in solution ($k_{on}\sim10^5$-$10^6 M^{-1}s^{-1}$) (Gao et al. (2006) Nucl. Acids Res. 34(11): 3370-3377).

The spacer bar can be regenerated effortlessly by reloading the sling with fresh antigen-modified c-spacer. Rinsing the surfaces with NaOH solution (pH13) for app. 10 seconds suffices to denature the double strand and remove bound c-spacer. After the pH13-treatment the handle refolds and the sling can be hybridized with new c-spacer, as can be seen in Figure 3F which depicts 16 regeneration cycles showing little signal degradation.

b) Analysis of analyte binding to the "slingshot layers"

To assess the selective binding of analytes to antigen-modified combination product, various binding states were analyzed, cf. Figure 4. Two small molecules biotin (bio) and digoxigenin (dig) were used as antigens, giving rise to three variants of the spacer bar between the sling and the c-spacer: bio-spacer-bio, dig-spacer-dig, and dig-spacer-bio. As analytes with one or two binding sites, anti-bio Fab, anti-dig Fab, and anti-bio IgG were used.

The binding states were analyzed with a time-resolved fluorescence (TRF) measurement that allows to gauge the size of bound analyte from the switching dynamics of the nucleic acid structure on the micro-second scale (Langer et al. (2013) Nature Commun. 4: 2099). Figure 4A shows TRF upward switching traces of nucleic acid structures before and after binding analytes of different sizes (Fab≈50kDa, Fab+Fab≈100kDa, Fab+IgG≈200kDa). Due to their greater hydrodynamic friction, larger analytes slow the switching dynamics to a greater extent and the TRF-curves are less steep. For straightforward analysis, the time-resolved curves are converted to single numbers representing the switching velocity: the 'Dynamic Response' (DR) parameter equates to the integral under the curves (cf. Fig. 4A) (Langer et al. (2013) Nature Commun. 4: 2099); it decreases when analytes slow down the switching dynamics of a nucleic acid structure layer.

Figure 4B exemplifies the sequential binding of anti-dig Fabs and anti-bio IgGs to a layer of nucleic acid structures loaded with bispecific dig/bio c-spacers. The analyte concentrations were chosen well above the dissociation constant (KD) to ensure full saturation of all surface binding sites ($K_D<0.1nM<<c_{Fab},=10nM$). As expected from their different sizes, the binding of full antibodies results in a greater signal change than the binding of Fabs. Because the association kinetics were limited by mass-transport, the transitions are quasi-linear.

A combinatorial set of experiments where monovalent (anti-dig Fab, anti-bio Fab) and bivalent (anti-bio IgG) analytes were bound to bispecific (dig/bio) or bivalent (bio/bio, dig/dig) spacer bars in different order was performed to assess the selectivity of interactions between the antibody and the nucleic acid structure. The schematics below Figure 4C-H illustrate the different binding situations. Distinct binding states can be discriminated by the DR value (switching velocity), which scales in magnitude with the total size of bound analyte ($\Delta DR_{Fab}<\Delta DR_{Fab+Fab}<\Delta DR_{Fab+IgG}$). The bivalent IgG is an exception (cf. Fig. 4G): although its molecular weight exceeds that of two Fabs, $\Delta DR_{IgG}$ is smaller than $\Delta DR_{Fab+Fab}$. This indicates that the hydrodynamic friction of a flexible construct where two Fabs are individually bound (Fig. 4E,F) to the sling is greater than that of a rigid structure where one IgG is connected to the nucleic acid structure by both arms (Fig. 4G). Overall, binding to the nucleic acid structure is found to be site-specific and does not depend on the succession of binding events.

Next the dissociation behavior of bivalent antibodies was investigated in order to determine if the combination product is a suitable means to realize well-defined, i.e. mono-phasic, unbinding kinetics. To this end layers of bivalent probes of the present invention were compared to the conventional situation where monovalent antigen probes are randomly located on the surface. The unbinding behavior from the conventional layers does not follow a simple mono-exponential time course but exhibits biphasic dissociation kinetics, in agreement with literature reports on bivalent binders studied with other biosensors (MacKenzie et al. (1996) J. Biol. Chem. 271(3): 1527-1533): SPR biphasic dissociation of dimeric scFv]. Accordingly, a double-exponential function of the form $(t)\propto A_1\cdot exp\{-k_{off,1}\cdot t\}+A_2.exp\{-k_{off,2}\cdot t\}$ is used to fit the data by nonlinear regression. Here, $k_{off,1}=10.0\ (2.0)\times10^{-4}$ and $k_{off,2}=6.3\ (0.3)\times10^{-5}$ are the dissociation rate constants of two independent dissociating species and the amplitudes $A_1$ and $A_2$ are their relative abundances on the sensor ($A_1+A_2=25+75=100\%$).

The two processes are dissociations of IgGs which are bound to one antigen (fast $k_{off,1}$) or two antigens (slow $k_{off,2}$), respectively. In the latter case the bivalent IgG must interlink two antigen modified probes within its reach. This could be confirmed in previous experiments where the relative amplitudes of the slow and fast dissociations were studied as a function of the density of antigen probes. The contribution from the slowly dissociating species was found to scale with the antigen density, verifying that the slow $k_{off,2}$ stems from IgGs which are doubly bound by interlinking.

In contrast, dissociation from bivalent combination products modified with two biotins proceeds in a simple, monophasic manner. A single-exponential function describes the data well, yielding a $k_{off}=3.7(0.1)\times10^{-5}$ which corresponds to $k_{off,2}$ in the case of randomly located monovalent probes.

c) Characterization of the "triangular arch" layers

The assembly of the triangular arch was characterized on a biosensor surface with the switchSENSE technique. The experiment was performed in buffer solution (40 mM NaCl, 10 mM PaPi, 0.05% Tween 20) at 25°C. The concentrations of solute connector strand and antigen modified spacer bar DNA was 200 nM, each. The hybridization of the connector strand to surface-immobilized handle 1/2 can be followed in real-time when incubating the single stranded handles with solute connectors, see figure 7a. As the connector binds to the handles and forms the double stranded strands, the fluorescence modulation amplitude $\Delta F$ increases as the efficiency of the orientation modulation by the applied electric field is higher for double stranded DNA compared to single stranded DNA (Rant et al. (2007) Proc. Natl.

Acad. Sci. USA 104(44): 17364-17369). The distance of the dyes at the top end of the handles to the gold surface increases at negative potentials during hybridization, shown by an increasing fluorescence intensity at -0.4V (solid symbols Figure **7a**).

The subsequent hybridization of the antigen modified spacer bars to the nucleic acid structure can be seen in figure **7b.** As the spacer bars hybridize to the single stranded part of the connector and form the double stranded spacer an increase of the fluorescence intensity at +0.4V can be observed, Figure **7b** open symbols, due to an increased sterical interaction of the fully formed nucleic acid structure with the surface. As the hybridization of the spacer bar DNA does not significantly alter the switching properties of the structure and thus the distance of the dye towards the metal surface, we do not observe a major change of fluorescence intensity as observed for the hybridization of the connector strand.

d) Analysis of analyte binding to the combination product

To prove the capability of the triangular arch structure to be used on the switchSENSE platform the association of an antibody to a antigen modified spacer bar was analyzed. The small molecule biotin (bio) was used as antigen and coupled to spacer 1.

Figure **8** exemplifies the binding of an anti-bio IgG to the biotin modified triangular arch structure. The experiment is performed in buffer solution (40 mM NaCl, 10 mM PaPi, 0.05% Tween 20) at 25°C. IgG solution of 16 nM concentration is injected at time t = 90 s. The antibody increases the overal hydrodynamic drag of the structure, slowing the switching motion. The change of dynamic response, as a measure of the switching speed, can be fitted by a single exponential decay, resulting in an association constant of $k_{on}$=1.8 (0.3)×$10^6$ M$^{-1}$s$^{-1}$.

**Claims**

1. A combination product comprising:

    (a) a nucleic acid structure having a sequence selected such that it forms at least one double strand (4) and at least one single-stranded loop (1); and
    (b)

        (i) one single-stranded nucleic acid molecule (6) which is capable of hybridizing to and forming a double strand with the single-stranded loop and to which a first ligand (7) is attached to the 5' end of the single-stranded nucleic acid molecule (6) and a second ligand (8) is attached to the 3' end of the single-stranded nucleic acid molecule (6); or
        (ii) a first (11) and a second (12) single-stranded nucleic acid molecule,

    wherein the first and the second nucleic acid molecules (11,12) are capable of hybridizing to and forming a double strand with different parts of the single-stranded loop (1) and wherein a first ligand (7) is attached to the first nucleic acid molecule (11) and a second ligand (8) is attached to the second nucleic acid molecule (12); wherein both the first and the second ligand (7,8) is a protein or a part thereof.

2. The combination product of claim 1, wherein the nucleic acid structure is a stem-loop structure.

3. A combination product comprising:

    (a) a nucleic acid structure formed by three separate single-stranded nucleic acid molecules, wherein the first (13) and the second (14) single-stranded nucleic acid molecule each form a double strand with a first and a second part of the third single-stranded nucleic acid molecule (15) and wherein a third part of the third single-stranded nucleic acid molecule which is located between the first and the second part forms a single-stranded loop; and

(b) at least one single-stranded nucleic acid molecule (6, 11, 12) which is capable of hybridizing to and forming a double strand with the single-stranded loop and to which at least one ligand (7,8) is attached,

wherein the at least one ligand (7,8) is a protein or a part thereof.

4. The combination product of claim 3, comprising one single-stranded nucleic acid molecule (6) which is capable of hybridizing to and forming a double strand with the single-stranded loop and to which a first ligand (7) is attached to the 5' end of the single-stranded nucleic acid molecule (6) and a second ligand (8) is attached to the 3' end of the single-stranded nucleic acid molecule (6).

5. The combination product of claim 3, comprising a first (11) and a second (12) single-stranded nucleic acid molecule, wherein the first and the second nucleic acid molecules (11,12) are capable of hybridizing to and forming a double strand with different parts of the single-stranded loop (1) and wherein a first ligand (7) is attached to the first nucleic acid molecule (11) and a second ligand (8) is attached to the second nucleic acid molecule (12).

6. The combination product of claim 1, 4 or 5, wherein the first ligand (7) and the second ligand (8) are the same.

7. The combination product of claim 1, 4 or 5, wherein the first ligand (7) and the second ligand (8) are different.

8. The combination product of any of the preceding claims, wherein the nucleic acid structure is attached to a substrate (10).

9. The combination product of any of the preceding claims, wherein the nucleic acid structure is labeled with a fluorescent dye (2).

10. A substrate to which at least one combination product of any of the preceding claims is attached.

11. A kit comprising the combination product of any of claims 1 to 9 or the substrate of claim 10 and instructions for its use.

12. Use of the combination product of any of claims 1 to 9 or of the substrate of claim 10 or of the kit of claim 11 for the detection and/or characterization of the interaction of a ligand with at least one binding partner thereof.

13. Use of the combination product of any of claims 1 to 9 or of the substrate of claim 10 or of the kit of claim 11 for protein purification or for the diagnosis of diseases.

14. Method for detecting and/or characterizing molecular interactions comprising:

(i) providing a combination product of any of claims 1, 2 and 6 to 9, wherein the single-stranded nucleic acid molecule (6) or the first and the second single-stranded molecule (11, 12) forms a double strand with the single-stranded loop; or
providing a combination product of any of claims 3 to 9, wherein the at least one single-stranded nucleic acid molecule (6, 11, 12) forms a double strand with the single-stranded loop;
(ii) contacting the combination product with a sample of the binding partner which is to be analyzed; and
(iii) detecting and/or characterizing the interaction between the ligand and the binding partner.

15. Method for detecting and/or characterizing molecular interactions comprising:

(i) contacting a single-stranded nucleic acid molecule (6) to which a first ligand (7) is attached to the 5' end of the single-stranded nucleic acid molecule (6) and a second ligand (8) is attached to the 3' end of the single-stranded nucleic acid molecule (6) or contacting a first (11) and a second (12) single-stranded nucleic acid molecule, wherein a first ligand (7) is attached to the first nucleic acid molecule (11) and a second ligand (8) is attached to the second nucleic acid molecule (12), with a sample of the binding partner which is to be analyzed, thereby providing a first mixture, wherein both the first and the second ligand (7,8) is a protein or a part thereof;
(ii) contacting the first mixture with the nucleic acid structure as described in claim 1 or 2, wherein the single-stranded molecule (6) is capable of hybridizing to and forming a double strand with the single-stranded loop (1) of the nucleic acid structure, and wherein the first and the second single-stranded nucleic acid molecules (11, 12) are capable of hybridizing to and forming a double strand with different parts of the single-stranded loop (1) of the nucleic acid structure, and allowing the single-stranded molecule(s) (6, 11, 12) to form said double strand with the single-stranded loop; and

(iii) detecting and/or characterizing the interaction between the ligand and the binding partner.

16. Method for detecting and/or characterizing molecular interactions comprising:

(i) contacting at least one single-stranded nucleic acid molecule (6, 11, 12) to which at least one ligand (7,8) is attached with a sample of the binding partner which is to be analyzed, thereby providing a first mixture, wherein the at least one ligand (7,8) is a protein or a part thereof;
(ii) contacting the first mixture with the nucleic acid structure as described in claim 3, wherein the at least one single-stranded nucleic acid molecule (6, 11, 12) is capable of hybridizing to and forming a double strand with the single-stranded loop of the nucleic acid structure, and allowing the single-stranded nucleic acid molecule to form said double strand with the single-stranded loop; and
(iii) detecting and/or characterizing the interaction between the ligand and the binding partner.

17. Method of any of claims 14 to 16, wherein the ligand is an antigen and the binding partner is an antibody.

18. Method of claim 17, wherein the binding partner is a bispecific antibody.

19. Method of any of claims 14 to 18, wherein the combination product is attached to a substrate, the substrate having at least one electrode, and wherein the combination product is labeled with a fluorescent dye and wherein the interaction is detected and/or **characterized by** the steps of:

(iv) applying an external electrical field to the electrode to cause a movement of said combination product;
(v) observing a signal of the label during said movement of the combination product,
(vi) using the observed signal for detecting an interaction of the binding partner with the at least one ligand.

20. Method for separating a target protein from impurities comprising the steps of:

(a) providing a substrate of claim 10, wherein the at least one single-stranded nucleic acid molecule (6, 11, 12) forms a double strand with the single-stranded loop;
(b) incubating the substrate with a sample containing the target protein under conditions allowing the target protein to bind to the ligand;
(c) washing the substrate under suitable conditions to remove impurities; and
(d) releasing the purified target protein from the substrate.

**Patentansprüche**

1. Kombinationsprodukt umfassend:

(a) eine Nukleinsäurestruktur, die eine Sequenz aufweist, die so ausgewählt ist, dass sie mindestens einen Doppelstrang (4) und mindestens eine einzelsträngige Schleife bildet (1); und
(b)

(i) ein einzelsträngiges Nukleinsäuremolekül (6), das in der Lage ist, mit der einzelsträngigen Schleife zu hybridisieren und damit einen Doppelstrang zu bilden und an das ein erster Ligand (7) am 5'-Ende des einzelsträngigen Nukleinsäuremoleküls (6) angeheftet ist und ein zweiter Ligand (8) an das 3'-Ende des einzelsträngigen Nukleinsäuremoleküls (6) angeheftet ist; oder
(ii) ein erstes (11) und ein zweites (12) einzelsträngiges Nukleinsäuremolekül, wobei die ersten und die zweiten Nukleinsäuremoleküle (11, 12) in der Lage sind, mit unterschiedlichen Teilen der einzelsträngigen Schleife (1) zu hybridisieren und damit einen Doppelstrang zu bilden und wobei ein erster Ligand (7) an das erste Nukleinsäuremolekül (11) angeheftet ist und ein zweiter Ligand (8) an das zweite Nukleinsäuremolekül (12) angeheftet ist;

wobei sowohl der erste als auch der zweite Ligand (7, 8) ein Protein oder ein Teil davon ist.

2. Kombinationsprodukt nach Anspruch 1, wobei die Nukleinsäurestruktur eine Stamm-Schleife-Struktur ist.

3. Kombinationsprodukt umfassend:

(1) eine Nukleinsäurestruktur, die durch drei unterschiedliche einzelsträngige Nukleinsäuremoleküle gebildet wird, wobei das erste (13) und das zweite (14) einzelsträngige Nukleinsäuremolekül jeweils einen Doppelstrang mit einem ersten und einem zweiten Teil des dritten einzelsträngigen Nukleinsäuremoleküls (15) bildet und wobei ein dritter Teil des dritten einzelsträngigen Nukleinsäuremoleküls, der sich zwischen dem ersten und dem zweiten Teil befindet, eine einzelsträngige Schleife bildet; und

(b) mindestens ein einzelsträngiges Nukleinsäuremolekül (6, 11, 12), das in der Lage ist, mit der einzelsträngigen Schleife zu hybridisieren und damit einen Doppelstrang zu bilden und an das mindestens ein Ligand (7, 8) angeheftet ist, wobei der mindestens eine Ligand (7, 8) ein Protein oder ein Teil davon ist.

4. Kombinationsprodukt nach Anspruch 3, umfassend ein einzelsträngiges Nukleinsäuremolekül (6), das in der Lage ist, mit der einzelsträngigen Schleife zu hybridisieren und damit einen Doppelstrang zu bilden, und an das ein erster Ligand (7) an das 5'-Ende des einzelsträngigen Nukleinsäuremoleküls (6) angeheftet ist und ein zweiter Ligand (8) an das 3'-Ende des einzelsträngigen Nukleinsäuremoleküls (6) angeheftet ist.

5. Kombinationsprodukt nach Anspruch 3, umfassend ein erstes (11) und ein zweites (12) einzelsträngiges Nukleinsäuremolekül, wobei die ersten und die zweiten Nukleinsäuremoleküle (11, 12) in der Lage sind, mit unterschiedlichen Teilen der einzelsträngigen Schleife (1) zu hybridisieren und damit einen Doppelstrang zu bilden, und wobei ein erster Ligand (7) an das erste Nukleinsäuremolekül (11) angeheftet ist und ein zweiter Ligand (8) an das zweite Nukleinsäuremolekül (12) angeheftet ist.

6. Kombinationsprodukt nach Anspruch 1, 4 oder 5, wobei der erste Ligand (7) und der zweite Ligand (8) gleich sind.

7. Kombinationsprodukt nach Anspruch 1, 4 oder 5, wobei der erste Ligand (7) und der zweite Ligand (8) unterschiedlich sind.

8. Kombinationsprodukt nach einem der vorhergehenden Ansprüche, wobei die Nukleinsäurestruktur an ein Substrat (10) angeheftet ist.

9. Kombinationsprodukt nach einem der vorhergehenden Ansprüche, wobei die Nukleinsäurestruktur mit einem Fluoreszenzfarbstoff (2) markiert ist.

10. Substrat, an das mindestens ein Kombinationsprodukt nach einem der vorhergehenden Ansprüche angeheftet ist.

11. Kit umfassend das Kombinationsprodukt nach einem der Ansprüche 1 bis 9 oder das Substrat nach Anspruch 10 und eine Gebrauchsanweisung.

12. Verwendung des Kombinationsprodukts nach einem der Ansprüche 1 bis 9 oder des Substrats nach Anspruch 10 oder des Kits nach Anspruch 11 zum Nachweis und/oder der Charakterisierung der Wechselwirkung eines Liganden mit mindestens einem seiner Bindungspartner.

13. Verwendung des Kombinationsprodukts nach einem der Ansprüche 1 bis 9 oder des Substrats nach Anspruch 10 oder des Kits nach Anspruch 11 zur Proteinaufreinigung oder zur Diagnose von Krankheiten.

14. Verfahren zum Nachweisen und/oder der Charakterisieren von molekularen Wechselwirkungen umfassend:

(i) Bereitstellen eines Kombinationsprodukts nach einem der Ansprüche 1, 2 und 6 bis 9, wobei das einzelsträngige Nukleinsäuremolekül (6) oder das erste und das zweite einzelsträngige Molekül (11, 12) einen Doppelstrang mit der einzelsträngigen Schleife bildet; oder
Bereitstellen eines Kombinationsprodukts nach einem der Ansprüche 3 bis 9, wobei das mindestens eine einzelsträngige Nukleinsäuremolekül (6, 11, 12) einen Doppelstrang mit der einzelsträngigen Schleife bildet;
(ii) Inkontaktbringen des Kombinationsprodukts mit einer Probe des Bindungspartners, der analysiert werden soll; und
(iii) Nachweisen und/oder Charakterisieren der Wechselwirkung zwischen dem Liganden und dem Bindungspartner.

15. Verfahren zum Nachweisen und/oder der Charakterisieren von molekularen Wechselwirkungen umfassend:

(i) Inkontaktbringen eines einzelsträngigen Nukleinsäuremoleküls (6), an das ein erster Ligand (7) am 5'-Ende des einzelsträngigen Nukleinsäuremoleküls (6) angeheftet ist und ein zweiter Ligand (8) an das 3'-Ende des einzelsträngigen Nukleinsäuremoleküls (6) angeheftet ist, oder Inkontaktbringen eines ersten (11) und eines zweiten (12) einzelsträngigen Nukleinsäuremoleküls, wobei ein erster Ligand (7) an das erste Nukleinsäuremolekül (11) angeheftet ist und ein zweiter Ligand (8) an das zweite Nukleinsäuremolekül (12) angeheftet ist, mit einer Probe des Bindungspartners, der analysiert werden soll, wodurch ein erstes Gemisch bereitgestellt wird, wobei sowohl der erste als auch der zweite Ligand (7, 8) ein Protein oder ein Teil davon ist;
(ii) Inkontaktbringen des ersten Gemisches mit der Nukleinsäurestruktur wie in Anspruch 1 oder 2 beschrieben, wobei das einzelsträngige Molekül (6) in der Lage ist, an die einzelsträngige Schleife (1) zu hybridisieren und damit einen Doppelstrang zu bilden, und wobei die ersten und die zweiten einzelsträngigen Nukleinsäuremoleküle (11, 12) in der Lage sind, mit unterschiedlichen Teilen der einzelsträngigen Schleife (1) zu hybridisieren und damit einen Doppelstrang zu bilden, und gestatten, dass das/die einzelsträngige(n) Molekül(e) (6, 11, 12) den Doppelstrang mit der einzelsträngigen Schleife bildet/bilden; und
(iii) Nachweisen und/oder Charakterisieren der Wechselwirkung zwischen dem Liganden und dem Bindungspartner.

16. Verfahren zum Nachweisen und/oder Charakterisieren molekularer Wechselwirkungen, umfassend:

(i) Inkontaktbringen von mindestens einem einzelsträngigen Nukleinsäuremolekül (6, 11, 12), an das mindestens ein Ligand (7, 8) angeheftet ist, mit einer Probe des Bindungspartners, der analysiert werden soll, wodurch ein erstes Gemisch bereitgestellt wird, wobei der mindestens eine Ligand (7, 8) ein Protein oder ein Teil davon ist;
(ii) Inkontaktbringen des ersten Gemisches mit der Nukleinsäurestruktur wie im Anspruch 3 beschrieben, wobei das mindestens eine einzelsträngige Nukleinsäuremolekül (6, 11, 12) in der Lage ist, an die einzelsträngige Schleife der Nukleinsäurestruktur zu hybridisieren und damit einen Doppelstrang zu bilden, und gestatten, dass das einzelsträngige Nukleinsäuremolekül den Doppelstrang mit der einzelsträngigen Schleife bildet, und
(iii) Nachweisen und/oder Charakterisieren der Wechselwirkung zwischen dem Liganden und dem Bindungspartner.

17. Verfahren nach einem der Ansprüche 14 bis 16, wobei der Ligand ein Antigen und der Bindungspartner ein Antikörper ist.

18. Verfahren nach Anspruch 17, wobei der Bindungspartner ein bispezifischer Antikörper ist.

19. Verfahren nach einem der Ansprüche 14 bis 18, wobei das Kombinationsprodukt an ein Substrat angeheftet ist, das mindestens eine Elektrode aufweist, und wobei das Kombinationsprodukt mit einem Fluoreszenzfarbstoff markiert ist und wobei die Wechselwirkung nachgewiesen und/oder charakterisiert wird durch die Schritte:

(iv) Anlegen eines externen elektrischen Feldes an die Elektrode, um eine Bewegung des Kombinationsprodukts auszulösen;
(v) Beobachten eines Signals der Markierung während der Bewegung des Kombinationsprodukts,
(vi) Verwenden des beobachteten Signals zum Nachweisen einer Wechselwirkung des Bindungspartners mit dem mindestens einen Liganden.

20. Verfahren zum Trennen eines Zielproteins von Verunreinigungen, umfassend die Schritte:

(a) Bereitstellen eines Substrats nach Anspruch 10, wobei das mindestens eine einzelsträngige Nukleinsäuremolekül (6, 11, 12) einen Doppelstrang mit der einzelsträngigen Schleife bildet;
(b) Inkubieren des Substrats mit einer Probe enthaltend das Zielprotein unter Bedingungen, die es dem Zielprotein erlauben, an den Liganden zu binden;
(c) Waschen des Substrats unter geeigneten Bedingungen, um Verunreinigungen zu entfernen; und
(d) Freisetzen des aufgereinigten Zielproteins von dem Substrat.

**Revendications**

1. Produit de combinaison, comprenant :

   (a) une structure d'acide nucléique présentant une séquence choisie de telle sorte qu'elle forme au moins une double hélice (4) et au moins une boucle simple brin (1) ; et
   (b)

   (i) une molécule d'acide nucléique simple brin (6) qui est capable de s'hybrider et de former une double hélice avec la boucle simple brin et à laquelle un premier ligand (7) est attaché à l'extrémité 5' de la molécule d'acide nucléique simple brin (6) et un second ligand (8) est attaché à l'extrémité 3' de la molécule d'acide nucléique simple brin (6) ; ou
   (ii) une première (11) et une seconde (12) molécule d'acide nucléique simple brin, les première et seconde molécules d'acide nucléique (11, 12) étant capables de s'hybrider et de former une double hélice avec différentes parties de la boucle simple brin (1) et un premier ligand (7) étant attaché à la première molécule d'acide nucléique (11) et un second ligand (8) étant attaché à la seconde molécule d'acide nucléique (12) ;

   dans lequel à la fois le premier et le second ligand (7, 8) est une protéine ou une partie de celle-ci.

2. Produit de combinaison selon la revendication 1, dans lequel la structure d'acide nucléique est une structure en boucle formant tige.

3. Produit de combinaison, comprenant :

   (a) une structure d'acide nucléique formée par trois molécules d'acide nucléique simple brin séparées, la première (13) et la deuxième (14) molécule d'acide nucléique simple brin formant chacune une double hélice avec une première et une deuxième partie de la troisième molécule d'acide nucléique simple brin (15) et une troisième partie de la troisième molécule d'acide nucléique simple brin qui est située entre la première et la deuxième parties formant une boucle simple brin ; et
   (b) au moins une molécule d'acide nucléique simple brin (6, 11, 12) qui est capable de s'hybrider et de former une double hélice avec la boucle simple brin et à laquelle au moins un ligand (7, 8) est attaché,

   dans lequel au moins un ligand (7, 8) est une protéine ou une partie de celle-ci.

4. Produit de combinaison selon la revendication 3, comprenant une molécule d'acide nucléique simple brin (6) qui est capable de s'hybrider et de former une double hélice avec la boucle simple brin et à laquelle un premier ligand (7) est attaché à l'extrémité 5' de la molécule d'acide nucléique simple brin (6) et un second ligand (8) est attaché à l'extrémité 3' de la molécule d'acide nucléique simple brin (6).

5. Produit de combinaison selon la revendication 3, comprenant une première (11) et une seconde (12) molécule d'acide nucléique simple brin, la première et la seconde molécule d'acide nucléique (11, 12) étant capables de s'hybrider et de former une double hélice avec différentes parties de la boucle simple brin (1) et un premier ligand (7) étant attaché à la première molécule d'acide nucléique (11) et un second ligand (8) étant attaché à la seconde molécule d'acide nucléique (12).

6. Produit de combinaison selon la revendication 1, 4 ou 5, dans lequel le premier ligand (7) et le second ligand (8) sont identiques.

7. Produit de combinaison selon la revendication 1, 4 ou 5, dans lequel le premier ligand (7) et le second ligand (8) sont différents.

8. Produit de combinaison selon une quelconque des revendications précédentes, dans lequel la structure d'acide nucléique est attachée à un substrat(10).

9. Produit de combinaison selon une quelconque des revendications précédentes, dans lequel la structure d'acide nucléique est marquée avec un colorant fluorescent (2).

10. Substrat auquel au moins un produit de combinaison selon une quelconque des revendications précédentes est attaché.

**11.** Kit comprenant le produit de combinaison selon une quelconque des revendications 1 à 9 ou le substrat selon la revendication 10 et des consignes pour l'utiliser.

**12.** Utilisation du produit de combinaison selon une quelconque des revendications 1 à 9 ou du substrat selon la revendication 10 ou du kit selon la revendication 11, pour la détection et/ou la caractérisation de l'interaction d'un ligand avec au moins un partenaire de liaison de celui-ci.

**13.** Utilisation du produit de combinaison selon une quelconque des revendications 1 à 9 ou du substrat selon la revendication 10 ou du kit selon la revendication 11, pour la purification de protéines ou pour le diagnostic de maladies.

**14.** Procédé pour la détection et/ou la caractérisation d'interactions moléculaires, consistant à :

(i) fournir un produit de combinaison selon une quelconque des revendications 1, 2 et 6 à 9, la molécule d'acide nucléique simple brin (6) ou la première et la seconde molécule simple brin (11, 12) formant une double hélice avec la boucle simple brin ; ou
fournir un produit de combinaison selon une quelconque des revendications 3 à 9, dans lequel l'au moins une molécule d'acide nucléique simple brin (6, 11, 12) forme une double hélice avec la boucle simple brin ;
(ii) mettre en contact le produit de combinaison avec un échantillon du partenaire de liaison qui est à analyser ; et
(iii) détecter et/ou caractériser l'interaction entre le ligand et le partenaire de liaison.

**15.** Procédé pour la détection et/ou la caractérisation d'interactions moléculaires, consistant à :

(i) mettre en contact une molécule d'acide nucléique simple brin (6) à laquelle un premier ligand (7) est attaché à l'extrémité 5' de la molécule d'acide nucléique simple brin (6) et un second ligand (8) est attaché à l'extrémité 3' de la molécule d'acide nucléique simple brin (6) ou mettre en contact une première (11) et une seconde (12) molécule d'acide nucléique simple brin, un premier ligand (7) étant attaché à la première molécule d'acide nucléique (11) et un second ligand (8) étant attaché à la seconde molécule d'acide nucléique (12), avec un échantillon du partenaire de liaison qui est à analyser, fournissant ainsi un premier mélange, dans lequel à la fois le premier et le second ligand (7, 8) est une protéine ou une partie de celle-ci,
(ii) mettre en contact le premier mélange avec la structure d'acide nucléique décrite dans la revendication claim 1 ou 2, la molécule simple brin (6) étant capable de s'hybrider et de former une double hélice avec la boucle simple brin (1) de la structure d'acide nucléique, et la première et la seconde molécule d'acide nucléique simple brin (11, 12) étant capables de s'hybrider et de former une double hélice avec différentes parties de la boucle simple brin (1) de la structure d'acide nucléique, et permettre à la (aux) molécule (s) simple brin (6, 11, 12) de former ladite double hélice avec la boucle simple brin ; et
(iii) détecter et/ou caractériser l'interaction entre le ligand et le partenaire de liaison.

**16.** Procédé pour la détection et/ou la caractérisation d'interactions moléculaires, consistant à :

(i) mettre en contact au moins une molécule d'acide nucléique simple brin (6, 11, 12) à laquelle au moins un ligand (7, 8) est attaché avec un échantillon du partenaire de liaison qui est à analyser, fournissant ainsi un premier mélange, dans lequel l'au moins un ligand (7, 8) est une protéine ou une partie de celle-ci ;
(ii) mettre en contact le premier mélange avec la structure d'acide nucléique décrite selon la revendication 13, l'au moins une molécule d'acide nucléique simple brin (6, 11, 12) étant capable de s'hybrider et de former une double hélice avec la boucle simple brin de la structure d'acide nucléique, et permettre à la molécule d'acide nucléique simple brin de former ladite double hélice avec la boucle simple brin ; et
(iii) détecter et/ou caractériser l'interaction entre le ligand et le partenaire de liaison.

**17.** Procédé selon une quelconque des revendications 14 à 16, dans lequel le ligand est un antigène et le partenaire de liaison est un anticorps.

**18.** Procédé selon la revendication 17, dans lequel le partenaire de liaison est un anticorps bispécifique.

**19.** Procédé selon une quelconque des revendications 14 à 18, dans lequel le produit de combinaison est attaché à un substrat, le substrat présentant au moins une électrode, et dans lequel le produit de combinaison est marqué avec un colorant fluorescent et dans lequel l'interaction est détectée et/ou **caractérisée par** les étapes consistant à :

(iv) appliquer un champ électrique externe sur l'électrode pour causer un déplacement dudit produit de combinaison ;

(v) observer un signal du marquage pendant ledit déplacement du produit de combinaison ;

(vi) utiliser le signal observé pour détecter une interaction du partenaire de liaison avec l'au moins un ligand.

20. Procédé pour séparer une protéine cible des impuretés, comprenant les étapes consistant à :

(a) fournir un substrat selon la revendication 10, dans lequel l'au moins une molécule d'acide nucléique simple brin (6, 11, 12) forme une double hélice avec la boucle simple brin ;

(b) incuber le substrat avec un échantillon contenant la protéine cible dans les conditions permettant à la protéine cible de se lier au ligand ;

(c) laver le substrat dans des conditions appropriées pour enlever les impuretés ; et

(d) libérer la protéine cible purifiée du substrat.

Figure 1

Figure 2a

Figure 2b

Figure 3

Figure 4

Figure 3

Figure 5

Figure 6

Figure 7

a)                                                    b)

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030219803 A

### Non-patent literature cited in the description

- **VANCOTT.** *Methods Mol. Med.,* 1999, vol. 17, 273-282
- **HEARTY et al.** *Methods Mol. Biol.,* 2012, vol. 907, 411-442
- **RANT et al.** *Nano Letters,* 2009, vol. 9, 1290-1295
- **KNEZEVIC et al.** *J. Am. Chem. Soc.,* 2012, vol. 134 (37), 15225-15228
- **LANGER et al.** *Nature Communications,* 2013, vol. 4, 2099
- Detection and Size Analysis of Proteins with Switchable DNA Layers. *Nano Letters,* 2009, vol. 9, 1290-1295
- **HEGNER et al.** *FEBS,* 1993, vol. 336 (3), 452-456
- **FUJIWARA et al.** *J. Immunol. Methods,* 1988, vol. 112, 77-83
- **PEETERS et al.** *J. Immunol. Methods,* 1989, vol. 120, 133-143
- **HERMANSON.** Bioconjugate Techniques. Elsevier, 2013
- **MARK.** Bioconjugation Protocols. Humana Press, 2011
- *J. Phys. Chem.,* 2005, vol. 122, 061103
- **IQBAL et al.** *Proc. Natl. Acad. Sci. USA,* 2008, vol. 105 (32), 11176-11181
- **ZUKER.** *Nucl. Acids Res.,* 2003, vol. 31 (13), 3406-3415
- **LANGER et al.** *Nature Commun.,* 2013, vol. 4, 2099
- **RANT et al.** *Proc. Natl. Acad. Sci. USA,* 2007, vol. 104 (44), 17364-17369
- **KAISER ; RANT.** *J. Am. Chem. Soc.,* 2010, vol. 132 (23), 7935-45
- **TAWA ; KNOLL.** *Nucl. Acids Res.,* 2004, vol. 32 (8), 2372-2377
- **GAO et al.** *Nucl. Acids Res.,* 2006, vol. 34 (11), 3370-3377
- **MACKENZIE et al.** *J. Biol. Chem.,* 1996, vol. 271 (3), 1527-1533